# FASCICULE DE BREVET EUROPEEN

(11) **EP 0 858 785 B1**
(45) Date de publication et mention de la délivrance du brevet: **21.05.2003**
(21) Numéro de dépôt: 98400214.7
(22) Date de dépôt: 03.02.1998
(51) Int. Cl.: A61F 2/08

(54) **Matériel pour la fixation d'un tendon de muscle sur un os**
Material zum Befestigen einer Muskelsehne auf einem Knochen
Material for a fixation of a tendon to a bone

(30) Priorité: 05.02.1997 FR 9701308
(43) Date de publication de la demande: 19.08.1998
(73) Titulaire: ETHICON, 92130 Issy les Moulineaux (FR)
(72) Inventeur: Iserin, Alain, 75007 Paris (FR); Walsch, Gilles, 69003 Lyon (FR)
(74) Mandataire: Texier, Christian

(56) Documents cités:
- DE-U- 8 701 123
- FR-A- 2 283 661
- FR-A- 2 703 239
- US-A- 5 013 316
- US-A- 5 370 661

## Description

La présente invention est relative à un matériel pour la fixation d'un tendon de muscle sur un os.

Elle trouve en particulier avantageusement application pour la fixation des muscles qui se trouvent sur la partie supérieure de l'épaule - appelés muscles de la coiffe des rotateurs - sur la tête de l'humérus.

A ce jour, la fixation d'un muscle sur un os s'effectue aux moyens de sutures.

Comme on le comprendra, une telle fixation est longue et fastidieuse.

Un but de l'invention est de pallier cet inconvénient.

Un exemple de matériel de sutures permettant un ancrage sur un os est notamment décrit dans le document US 5 370 661.

On connaît par ailleurs par FR 2 283 661 des ligaments prothétiques qui permettent de réunir d'une manière flexible des éléments de squelette.

On connaît en outre par FR 2 703 239 des agrafes pour prothèses interépineuses qui comportent une tige, une tête de fixation et un élément de verrouillage qui coopère avec la tige à son extrémité qui est opposée à la tête de fixation qui est destinée à bloquer ladite tige et sa tête par rapport à un coussinet de prothèse interépineuse sur lequel l'ensemble de ce matériel est monté. Le document US-A-5 013 316 divulgue un matériel de fixation comme décrit dans le préambule de la revendication 1.

L'invention propose quant à elle un matériel de fixation comportant une tige, une tête de fixation qui termine ladite tige et un élément de verrouillage apte à coopérer avec la tige à son extrémité qui est opposée à la tête de fixation, caractérisé en ce que la tige se termine par une rotule sur laquelle la tête de fixation est articulée, afin de permettre la fixation d'un tendon de muscle sur un os, la tige étant destinée à traverser le tendon et un canal préalablement foré dans l'os et débouchant à ses deux extrémités, la tête de fixation étant destinée à venir en appui sur le muscle après avoir été orientée, pour maintenir ledit muscle contre l'os, l'élément de verrouillage étant destiné à coopérer avec la tige pour bloquer celle-ci et sa tête par rapport à l'os et au muscle.

Ce matériel est avantageusement complété par les différentes caractéristiques suivantes, prises seules ou selon toutes leurs combinaisons techniquement possibles :
- pour sa coopération avec l'élément de verrouillage, la tige présente une pluralité de crans répartis sur au moins une partie de sa longueur ;
- la tige présente une pluralité de zones crantées alternant avec des zones lisses ;
- l'élément de verrouillage est un tube qui est extérieurement de forme générale tronconique et qui est destiné à se fixer sur l'os par serrage de sa paroi tronconique dans le canal foré dans l'os ;
- l'élément de verrouillage présente une pluralité de crans internes destinés à coopérer avec les crans portés par la tige pour permettre le coulissement de l'élément de verrouillage le long de la tige dans un sens et s'opposer à un coulissement en sens contraire ;
- l'élément de verrouillage présente des évidements annulaires au droit des crans internes ;
- la tête présente au moins une pointe pour son ancrage dans le muscle ;
- la tête présente deux oreilles de part et d'autre d'une zone centrale, chaque oreille portant une pointe d'ancrage.

D'autres caractéristiques et avantages de l'invention ressortiront encore de la description qui suit. Cette description est purement illustrative et non limitative. Elle doit être lue en regard des dessins annexés sur lesquels :
- la figure 1 est une vue en plan d'un matériel de fixation conforme à un mode de réalisation possible de l'invention ;
- la figure 2 est une représentation en deux parties de la tige du matériel de la figure 1 ;
- la figure 3 est une vue de dessus de la tête de fixation du matériel de la figure 1 ;
- la figure 4 est une vue en coupe de la tête de fixation de la figure 3 ;
- la figure 5 est une vue en coupe de l'élément de verrouillage du matériel de la figure 1.

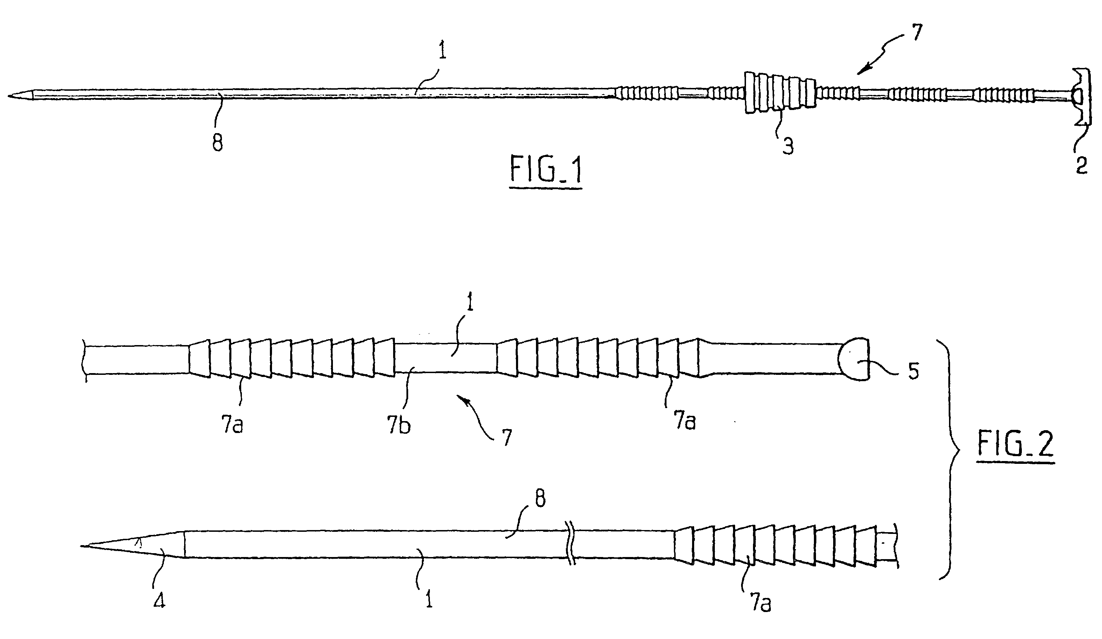

Le matériel de fixation illustré sur la figure 1 comporte une tige 1, une tête de fixation 2, ainsi qu'un élément de verrouillage 3.

La tige 1, qui a été plus particulièrement illustrée sur la figure 2, présente un corps cylindrique qui se termine à une extrémité par une pointe 4 et à une autre extrémité par une rotule 5 de forme semi-sphérique (sphère coupée par un méplat perpendiculaire à l'axe de la tige 1).

Le corps de ladite tige 1 présente sur une partie de sa longueur, du côté de la rotule 5 (partie référencée par 7 sur les figures), une pluralité de zones crantées 7a réparties régulièrement et séparées par des zones lisses 7b.

Les zones crantées 7a sont définies par des successions de dents de forme tronconiques qui s'évasent en direction de la rotule 5.

Du côté de la pointe 4, la tige 1 se termine par une partie lisse 8.

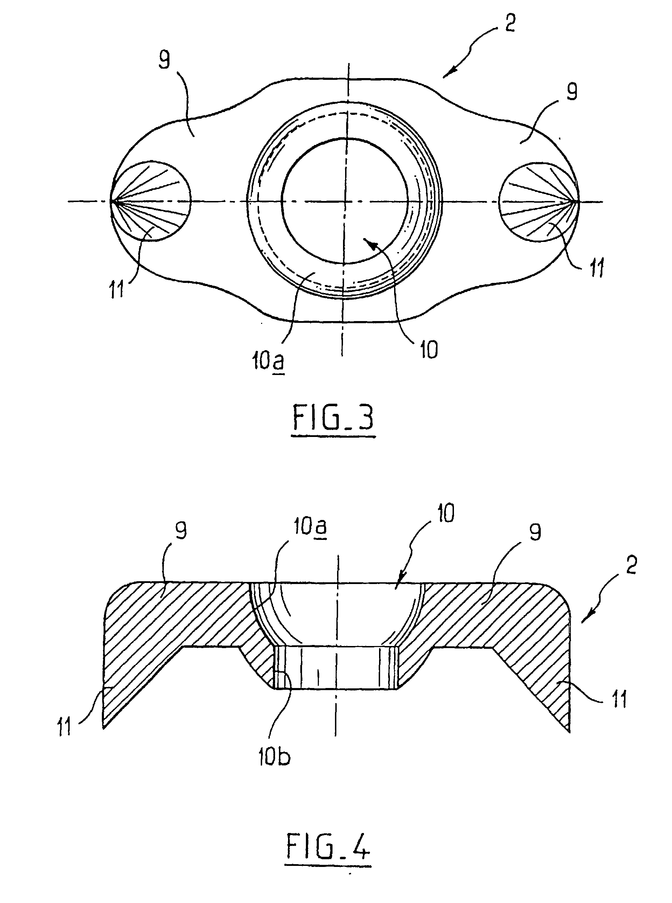

La tête de fixation 2 (figures 3 et 4) présente une forme allongée définie par deux oreilles 9, de forme plane, disposées de part et d'autre d'une zone centrale 10.

La zone centrale 10 est traversée par un orifice qui présente une portion 10a de forme semi-sphérique destinée à coopérer avec la rotule 5 pour permettre l'articulation de la tête de fixation 2 par rapport à la tige 1. Cette zone centrale comporte également une portion cylindrique 10b qui s'étend à partir du fond de la portion 10a.

Cette tête de fixation 2 est destinée à être enfilée sur la tige 1, par la pointe 4 de celle-ci,et à être remontée de façon que la portion 10a vienne coiffer la rotule 5.

Le diamètre intérieur de la portion 10b est choisi avec un jeu suffisant par rapport au diamètre de la tige 1 et aux dimensions des dents des zones crantées 7a pour permettre la remontée de la tête 2 le long de la tige 1, ainsi que le réglage de l'orientation de ladite tête 2 par rapport à la tige 1.

Chacune des oreilles planes 9 porte, sur sa face qui est du côté du corps de la tige 1 lorsque la tête 2 est en place sur ladite tige 1, une pointe 11 d'ancrage de forme conique.

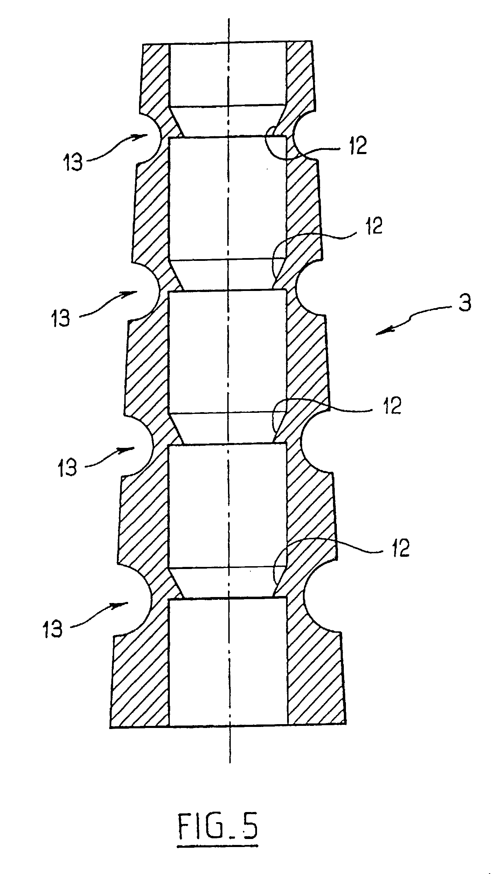

L'élément de verrouillage 3 (figure 5) est constitué par un tube de forme intérieure principalement cylindrique et de forme générale extérieure légèrement tronconique. Cet élément de verrouillage 3 présente en son intérieur une pluralité de crans 12 destinés à coopérer avec les crans de la tige 1.

Ces crans 12 sont des jupes élastiques de forme tronconique, qui sont destinées à s'effacer élastiquement lorsque l'élément de verrouillage est remonté sur la tige 1 et à s'opposer à des efforts qui tendraient à faire redescendre ledit élément de verrouillage 3 le long de ladite tige 1.

Le pas des crans 12 est le double du pas des crans des zones 7a.

Des évidements annulaires 13 semi toriques sont prévus sur la face extérieure de cet élément de verrouillage 3, au droit des jupes 12 pour conférer à celle-ci une élasticité maximale.

On donne ci-après un exemple de dimensionnement possible pour la tige 1, la tête 2 et l'élément de verrouillage 3.

Le diamètre des parties lisses de la tige 1 peut être de 1,5 mm.

La partie lisse 8 peut être d'une longueur de 95 mm, la partie 7 crantée étant de 70 mm.

Les zones crantées 7a de la partie 7 peuvent être d'une hauteur de 10 mm, les zones lisses 7b avec lesquelles elles alternent étant quant à elles d'une hauteur de 5 mm.

Le diamètre de la grande base des dents de la zone 7a est de 2 mm.

La rotule 5 est par exemple d'un diamètre de 2,25 mm et d'une hauteur de 1,5 mm.

Le diamètre intérieur de l'orifice 10b de la tête 2 est de 1,6 mm.

Les pointes 11 sont d'une hauteur de 1 mm, les oreilles 9 étant d'une épaisseur de 0,8 mm.

Le diamètre interne de l'élément 3 est de 2 mm, sa hauteur étant de 11 mm, son diamètre externe variant de 4 à 2,9 mm.

Les crans tronconiques 12 sont par exemple d'une hauteur de 0,5 mm, la distance qui sépare les extrémités de deux crans 12 successifs étant par exemple de 2,5 mm.

Les évidements annulaires 13, au nombre de quatre, présentent - en section droite par des plans passant par l'axe de l'élément 3 - des formes de demi-cercle dont le rayon varie de 0,6 à 0,4 mm.

Les matériaux de la tige 1, de la tête 2 et de l'élément de verrouillage 3 sont résorbables (PLLA ou PGA par exemple).

L'utilisation du matériel qui vient d'être décrit est par exemple la suivante.

Le chirurgien prépare l'os sur lequel le muscle doit être fixé en y forant un tunnel transosseux, dont le diamètre intérieur est par exemple, pour les dimensionnements de matériel précités, de 3,2 mm.

La tête de fixation 2 étant préalablement mise en place sur la tige 1, celle-ci est introduite dans le susépineux puis dans le tunnel préparé par le chirurgien dans l'os.

Le chirurgien oriente la tête de fixation 2 en fonction de la prise qu'il souhaite réaliser sur le muscle et positionne ladite tête de façon à ce qu'elle maintienne le muscle sur l'os.

On passe ensuite sur la tige 1, par son extrémité qui émerge du tunnel transosseux, l'élément de verrouillage 3, que l'on remonte sur la tige 1 pour la faire entrer dans le tunnel transosseux et réaliser un serrage de ces parois tronconiques contre l'os.

La pression de serrage de l'élément 3 contre l'os est par exemple contrôlée par un outil prévu à cet effet.

La partie de la tige 1 qui dépasse par rapport à l'élément de verrouillage est ensuite sectionnée.

## Revendications

1. Matériel de fixation comportant une tige (1), une tête de fixation (2) qui termine ladite tige (1) et un élément de verrouillage (3) apte à coopérer avec la tige (1) à son extrémité qui est opposée à la tête de fixation (2), **caractérisé en ce que** la tige se termine par une rotule (5) sur laquelle la tête de fixation (2) est articulée, afin de permettre la fixation d'un tendon de muscle sur un os, la tige étant destinée à traverser le tendon et un canal préalablement foré dans l'os et débouchant à ses deux extrémités, la tête de fixation étant destinée à venir en appui sur le muscle après avoir été orientée, pour maintenir ledit muscle contre l'os, l'élément de verrouillage étant destiné à coopérer avec la tige pour bloquer celle-ci et sa tête par rapport à l'os et au muscle.

2. Matériel selon la revendication 1, **caractérisé en ce que**, pour sa coopération avec l'élément de verrouillage (3), la tige (1) présente une pluralité de crans (7a) répartis sur au moins une partie de sa longueur.

3. Matériel selon la revendication 2, **caractérisé en ce que** la tige (1) présente une pluralité de zones crantées (7a) alternant avec des zones lisses (7b).

4. Matériel selon l'une des revendications précédentes, **caractérisé en ce que** l'élément de verrouillage (3) est un tube qui est extérieurement de forme générale tronconique et qui est destiné à se fixer sur l'os par serrage de sa paroi tronconique dans le canal foré dans l'os.

5. Matériel selon la revendication 4 prise en combinaison avec l'une des revendications 2 et 3, **caractérisé en ce que** l'élément de verrouillage (3) présente une pluralité de crans internes (12) destinés à coopérer avec les crans (7a) portés par la tige (1) pour permettre le coulissement de l'élément de verrouillage (3) le long de la tige (1) dans un sens et s'opposer à un coulissement en sens contraire.

6. Matériel selon la revendication 5,**caractérisé en ce que** l'élément de verrouillage (3) présente des évidements annulaires (13) au droit des crans internes (12).

7. Matériel selon l'une des revendications précédentes, **caractérisé en ce que** la tête (2) présente au moins une pointe (11) pour son ancrage dans le muscle.

8. Matériel selon la revendication 7, **caractérisé en ce que** la tête (2) présente deux oreilles (9) de part et d'autre d'une zone centrale (10), chaque oreille portant une pointe d'ancrage (11).

## Patentansprüche

1. Befestigungsmaterial, umfassend einen Stift (1), einen Befestigungskopf (2), der sich am Ende des Stifts (1) befindet, und ein Verriegelungselement (3), das dazu geeignet ist, mit dem Stift (1) an dessen Ende, das dem Befestigungskopf (2) gegenüberliegt, zusammenzuwirken, **dadurch gekennzeichnet, dass** der Stift in einem Kugelgelenk (5) endet, auf dem der Befestigungskopf (2) gelenkig angebracht ist, um die Befestigung einer Muskelsehne auf einem Knochen zu ermöglichen, wobei der Stift dazu bestimmt ist, die Sehne und einen Kanal, der vorab in dem Knochen gebohrt worden ist und an dessen beiden Enden mündet, zu durchdringen, wobei der Befestigungskopf dazu bestimmt ist, auf dem Muskel aufzuliegen, nachdem er orientiert worden ist, um den Muskel gegen den Knochen zu halten, wobei das Verriegelungselement dazu bestimmt ist, mit dem Stift zusammenzuwirken, um jenen und dessen Kopf bezogen auf den Knochen und den Muskel zu blockieren.

2. Material nach Anspruch 1, **dadurch gekennzeichnet, dass** für sein Zusammenwirken mit dem Verriegelungselement (3) der Stift (3) eine Mehrzahl von Einkerbungen (7a) aufweist, die über wenigstens einen Teil seiner Länge verteilt sind.

3. Material nach Anspruch 2, **dadurch gekennzeichnet, dass** der Stift (1) eine Mehrzahl von eingekerbten Bereichen (7a) aufweist, die mit glatten Bereichen (7b) alternieren.

4. Material nach einem der vorangegangenen Ansprüche, **dadurch gekennzeichnet, dass** das Verriegelungselement (3) ein Rohr ist, das äußerlich von allgemeiner kegelstumpfartiger Form ist und dazu bestimmt ist, sich an dem Knochen durch Einklemmen von seiner kegelstumpfartigen Wand in dem in den Knochen gebohrten Kanal zu fixieren.

5. Material nach Anspruch 4 zusammen genommen mit einem der Ansprüche 2 und 3, **dadurch gekennzeichnet, dass** das Verriegelungselement (3) eine Mehrzahl von inneren Einkerbungen (12) aufweist, die dazu bestimmt sind, mit den Einkerbungen (7a), die sich auf dem Stift (1) befinden, zusammenzuwirken, um das Gleiten des Verriegelungselements (3) entlang des Stifts (1) in einer Richtung zu ermöglichen und einem Gleiten in der entgegengesetzten Richtung entgegenzuwirken.

6. Material nach Anspruch 5, **dadurch gekennzeichnet, dass** das Verriegelungselement (3) ringförmige Vertiefungen (13) in einer Linie mit den inneren Einkerbungen (12) aufweist.

7. Material nach einem der vorangegangenen Ansprüche, **dadurch gekennzeichnet, dass** der Kopf (2) wenigstens eine Spitze (11) für seine Verankerung im Muskel aufweist.

8. Material nach Anspruch 7, **dadurch gekennzeichnet, dass** der Kopf (2) zwei Flügel beiderseits eines zentralen Bereichs (10) aufweist, wobei jeder Flügel eine Verankerungsspitze (11) trägt.

## Claims

1. A fastener comprising a shank (1), a fastening head (2) which terminates said shank (1), and a locking member (3) suitable for co-operating with the shank (1) at its end remote from the fastening head (2), the fastener being **characterized in that** the shank is terminated by a ball (5) on which the fastening head (2) is swivel-mounted so as to enable a muscle tendon to be fastened to a bone, the shank being designed to be inserted through the tendon and through a channel bored through the bone, the fastening head being designed to come into abutment against the muscle after being oriented to hold said muscle against the bone, the locking member being designed to co-operate with the shank to block said shank and its head relative to the bone and the muscle.

2. A fastener according to claim 1, **characterized in that**, to co-operate with the locking element (3), the shank (1) is provided with a plurality of serrations (7a) distributed uniformly over at least a portion of its length.

3. A fastener according to claim 2, **characterized in that** the shank (1) is provided with a plurality of serrated zones (7a) disposed in alternation with smooth zones (7b).

4. A fastener according to any preceding claim, **characterized in that** the locking element (3) is a tube which is substantially frustoconical externally, and which is designed to be fastened to the bone by means of its frustoconical wall being clamped in the channel bored through the bone.

5. A fastener according to claim 4, taken in association with claim 2 or 3, **characterized in that** the locking element (3) is provided with a plurality of internal serrations (12) designed to co-operate with the serrations (7a) carried by the shank (1) to enable the locking element (3) to slide along the shank (1) in one direction, and to prevent it from sliding therealong in the other direction.

6. A fastener according to claim 5, **characterized in that** the locking element (3) is provided with annular recesses (13) level with the internal serrations (12).

7. A fastener according to any preceding claim, **characterized in that** the head (2) is provided with at least one spike (11) for anchoring it in the muscle.

8. A fastener according to claim 7, **characterized in that** the head (2) is provided with two lugs (9) on respective sides of a central zone (10), each lug carrying an anchoring spike (11).
